# FASCICULE DE BREVET EUROPEEN

(11) **EP 1 064 959 B1**
(45) Date de publication et mention de la délivrance du brevet: **30.05.2012**
(21) Numéro de dépôt: 00401769.5
(22) Date de dépôt: 21.06.2000
(51) Int. Cl.: A61M 1/02, A61J 1/00, B01L 3/00, C12M 1/00

(54) **Ensemble de poches destiné à recevoir un fluide biologique et à mettre en évidence une éventuelle contamination du fluide biologique**
Beutelsatz zur Aufnahme von einer biologischen Flüssigkeit und zur Bestimmung von Kontamination der Flüssigkeit
Bag set for receiving a biological fluid and testing it for contamination

(30) Priorité: 30.06.1999 FR 9908383
(43) Date de publication de la demande: 03.01.2001
(73) Titulaire: Maco Pharma S.A., 59420 Mouvaux (FR)
(72) Inventeur: Grosdhomme, François, 60173 Ivry Le Temple (FR); Verpoort, Thierry, 59420 Mouvaux (FR)
(74) Mandataire: Herrou, Nathalie

(56) Documents cités:
- EP-A- 0 930 079
- WO-A-96/37177
- FR-A- 2 435 524
- US-A- 2 950 716
- US-A- 5 167 656
- US-A- 5 356 373

## Description

L'invention est relative aux tests de stérilité/contamination d'un fluide biologique tel que le sang ou un composant du sang.

Elle s'applique typiquement au cas où un tel fluide biologique est prélevé sur un donneur et est destiné à être envoyé vers et être reçu dans un système à poche(s). Un tel système à poche(s) comprend typiquement une ou plusieurs poches sur lesquelles sont branchées une ou plusieurs tubulures -notamment au moins une tubulure principale-, des moyens de dérivation et/ou d'arrêt associés à la ou aux tubulures, et un ensemble de prélèvement ou d'amenée du fluide biologique branché sur la tubulure principale.

Un des problèmes qui est posé avec ce type de système à poche(s) de réception d'un fluide biologique prélevé est le contrôle de la stérilité du dit fluide biologique.

On connaît déjà des systèmes à poche(s) qui sont mis en oeuvre de manière que les premiers millilitres de fluide prélevés soient éliminés étant donné qu'ils peuvent être l'objet d'une contamination de la barrière cutanée.

Mais ce procédé ne permet pas un contrôle de stérilité systématique.

Le document WO-96/37177 décrit un dispositif pour la détection d'un contaminant qui comprend une chambre primaire de stockage du fluide et une chambre secondaire de détection, un chemin de fluide étant prévu entre lesdites chambres.

Dans un tel dispositif, le test de contamination est réalisé avec du fluide provenant de la chambre primaire.

La réalisation du test est alors effectuée postérieurement au prélèvement du fluide et après que la chambre primaire a reçu le fluide.

L'invention vise à résoudre ces inconvénients au moyen d'une poche test contenant un milieu de culture ou un réactif formant avec le système à poche(s) un système clos et unitaire, dans lequel le test est réalisé avant ou sensiblement en même temps que le prélèvement du fluide.

A cet effet, et selon un premier aspect, la demande décrit application d'au moins une poche - dite poche test - à un système à poche(s) comprenant au moins une poche de manière que l'ensemble poche test - système à poche(s) soit clos et unitaire, la poche du système à poche(s) étant destinée à recevoir un fluide biologique - par exemple du sang ou un composant du sang -, la poche test étant, dans l'application, reliée à la poche par une ou un système de tubulures, la poche test étant apte à recevoir du fluide biologique à tester, la poche test contenant un milieu de culture ou un réactif qui recevant du fluide biologique est apte à mettre en évidence une éventuelle contamination du fluide biologique, dans laquelle le fluide biologique reçu dans la poche test a la même origine que celui ayant à être reçu dans le système à poche(s), tout en étant différent de lui pour ne pas provenir de la poche du système à poche(s).

Le fluide biologique reçu dans la poche test et le fluide biologique ayant à être reçu dans le système à poche(s) proviennent, par exemple, d'un même donneur, mais de façon séquentielle.

Selon un deuxième aspect, la demande décrit une poche test destinée à la réalisation d'un test de contamination destiné à mettre en évidence une éventuelle contamination d'un fluide biologique - par exemple du sang ou un composant du sang -, la dite poche test étant destinée à être appliquée de la manière décrite ci-dessus à un système à poche(s) lui-même destiné à recevoir le fluide biologique, la poche test étant destiné en premier lieu à contenir un milieu de culture ou un réactif qui recevant du fluide biologique est apte à mettre en évidence une éventuelle contamination du fluide biologique ; en deuxième lieu à recevoir du fluide biologique à tester ; et en troisième lieu à être reliée à une poche du système à poche(s) par une ou un système de tubulures.

La poche test est apte à contenir une quantité suffisante de milieu de culture ou de réactif et de fluide biologique pour la réalisation du test de contamination, par exemple de l'ordre de quelques millilitres de milieu de culture ou de réactif et de l'ordre de quelques millilitres de fluide biologique.

Selon un troisième aspect la demande décrit un ensemble comprenant une poche test telle qu'elle vient d'être décrite, le milieu de culture ou le réactif qu'elle contient et/ou la ou le système de tubulures qui relie la poche test à la poche du système à poche(s). La ou le système de tubulures comprenant également des moyens de dérivation et/ou d'arrêt commandés par des moyens d'actionnement.

Selon un quatrième aspect, la demande décrit un système à poche(s) destiné à recevoir un fluide biologique - par exemple du sang ou un composant du sang-, comprenant typiquement une ou plusieurs poches sur lesquelles sont branchées une ou plusieurs tubulures -notamment au moins une tubulure principale-, des moyens de dérivation et/ou d'arrêt associés à la ou aux tubulures, et un ensemble de prélèvement ou d'amenée du fluide biologique branché sur la tubulure principale, au moins une poche test lui étant appliquée telle que décrit ci-dessus, cette poche test étant, dans cette application, reliée à une poche du système à poche(s) par une ou un système de tubulures.

Selon un cinquième aspect, la demande décrit un ensemble système à poche(s) / poche test comprenant un système à poche(s) tel que décrit ci-dessus et au moins une poche test telle que décrite ci-dessus et/ou au moins un ensemble comprenant une poche test tel que décrit ci-dessus.

La ou le système de tubulures est branché sur une tubulure principale, elle-même branchée sur la poche du système à poche(s).

L'invention définit un ensemble système système à poches/poche test comme défini dans les revendications 1-7. Selon un deuxième aspect, l'invention vise un procédé de mise en oeuvre d'un ensemble système à poche(s) / poche test tel que défini dans les revendications 8-11.

Dans un premier mode d'exécution, le procédé comprend les étapes dans lesquelles :
- on prélève le fluide biologique à l'aide de l'ensemble de prélèvement ou d'amenée ;
- on dirige du fluide biologique vers la poche test jusqu'à ce qu'une quantité suffisante au test de contamination y soit introduite ;
- puis on actionne les moyens de dérivation et/ou d'arrêt pour fermer l'alimentation en fluide biologique de la poche test ;
- on dirige le fluide biologique vers la poche du système à poche(s) par l'intermédiaire de la tubulure principale.

Le fluide biologique peut être dirigé vers la poche test soit en même temps que vers la poche du système à poche(s) soit avant que vers la poche du système à poche(s).

La quantité de fluide biologique prélevée est alors suffisante pour la réalisation du test de contamination, par exemple de l'ordre de quelques millilitres de fluide biologique, pour l'introduire dans la poche test.

D'autres modes de réalisation et avantages de l'invention apparaîtront au cours de la description qui suit en référence aux dessins annexés, illustrant différents modes de réalisation.

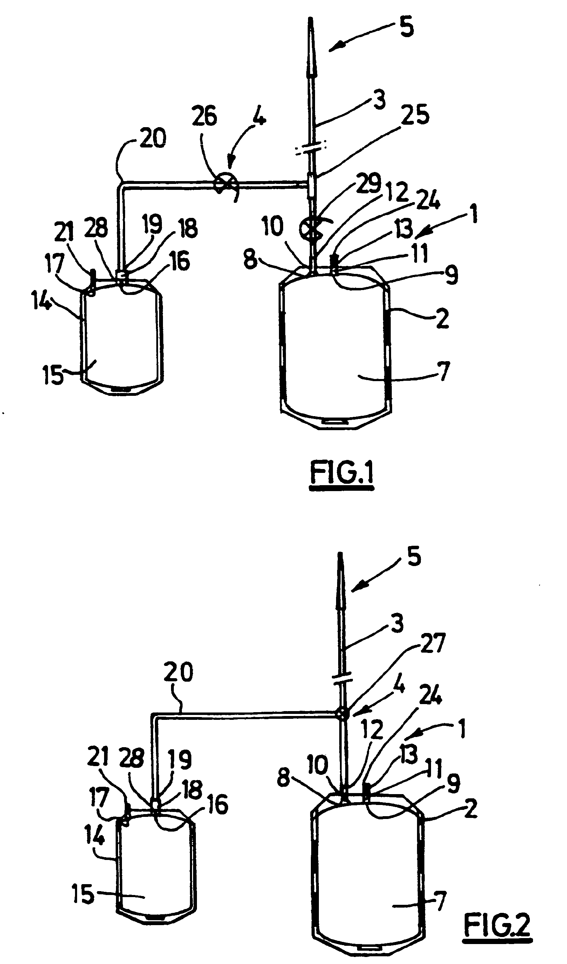

Les figures 1 et 2 sont des vues schématiques en élévation et à plat d'un premier et d'un deuxième mode de réalisation d'un ensemble comprenant un système à poche(s) et une poche test pour la réalisation d'un test de contamination du fluide biologique ayant à être reçu dans une poche du système à poche(s).

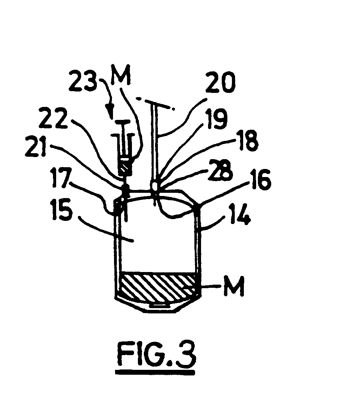

La figure 3 est une vue schématique d'une poche test représentée à plat et d'une seringue d'injection associée illustrant l'introduction du milieu de culture ou du réactif dans la poche test, selon la réalisation correspondante.

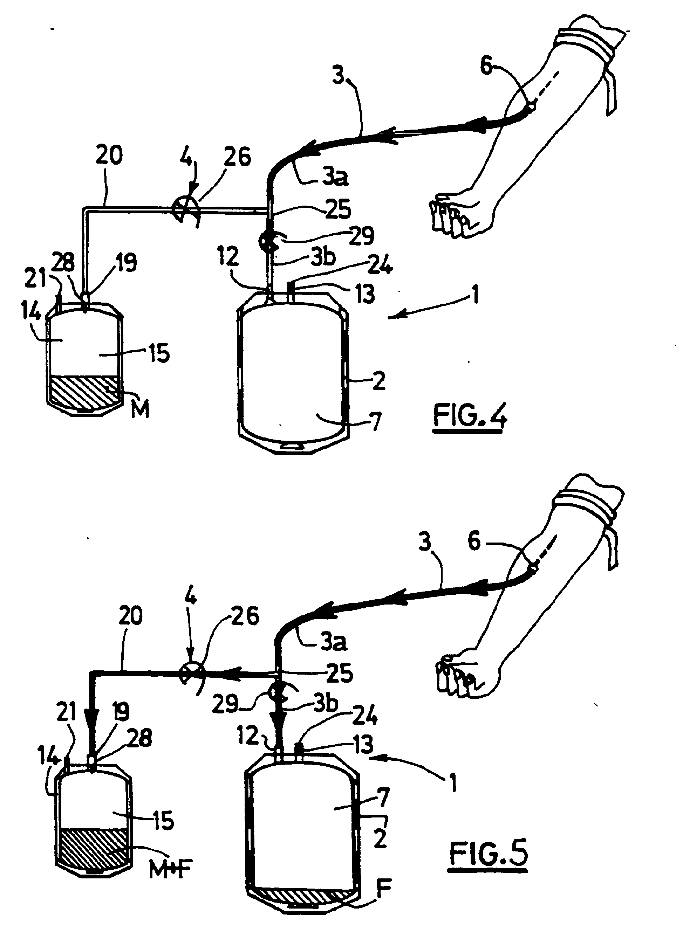

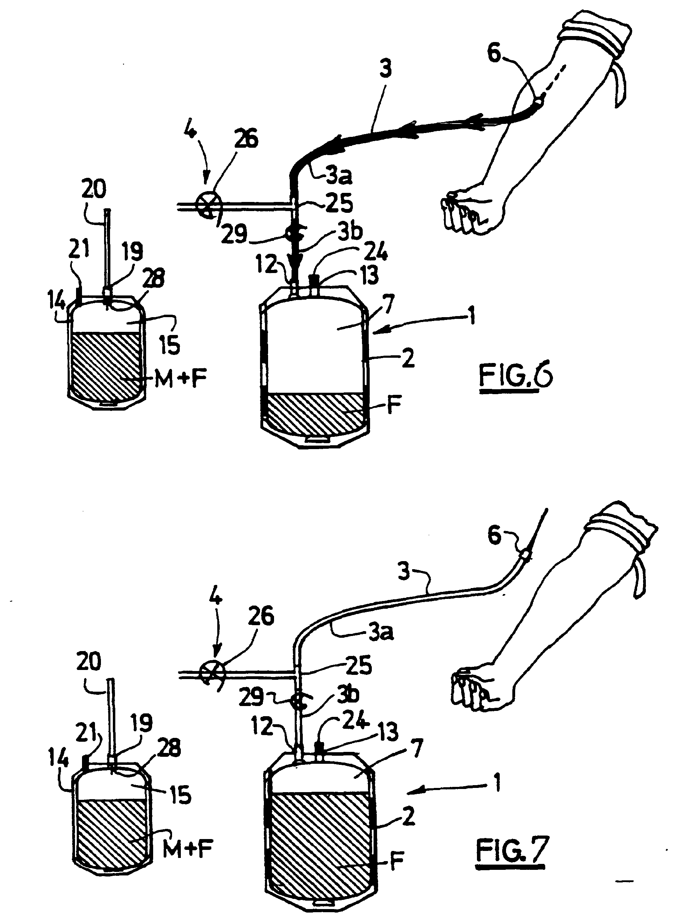

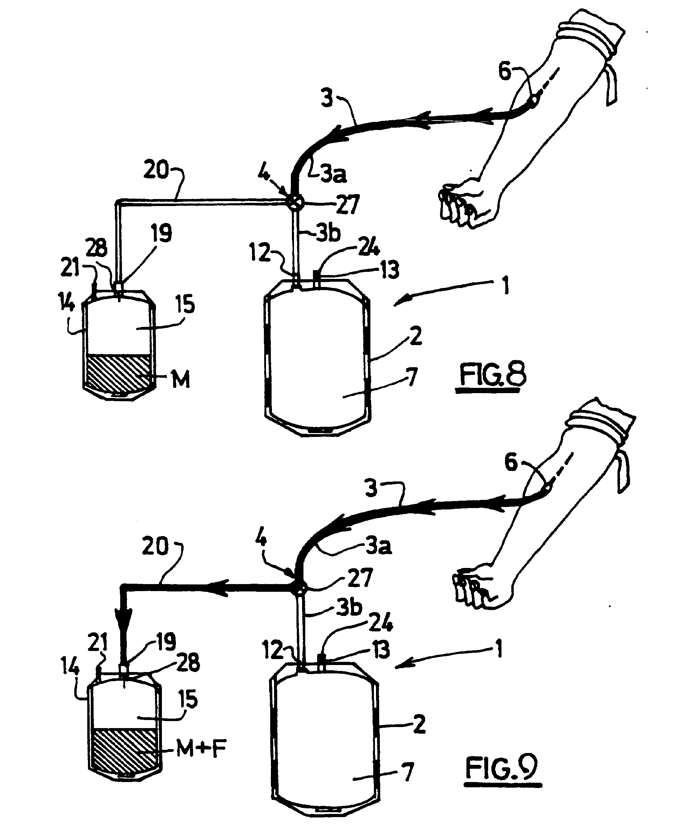

Les figures 4 à 7 sont des vues schématiques en élévation et à plat illustrant les étapes successives d'un mode d'exécution du procédé de mise en oeuvre du premier mode de réalisation représenté sur la figure 1. A savoir, pour la figure 5, l'étape de prélèvement du fluide biologique à l'aide de l'ensemble de prélèvement ou d'amenée ; pour la figure 6, l'alimentation simultanée de la poche du système à poche(s) et de la poche test ; pour la figure 7, une quantité suffisante de fluide biologique ayant été introduite dans la poche test, les moyens de dérivation et/ou d'arrêt sont actionnés pour permettre l'alimentation de la poche du système à poche(s) seule ; pour la figure 8, la quantité souhaitée de fluide biologique ayant été introduite dans la poche du système à poche(s), arrêt du prélèvement du fluide biologique.

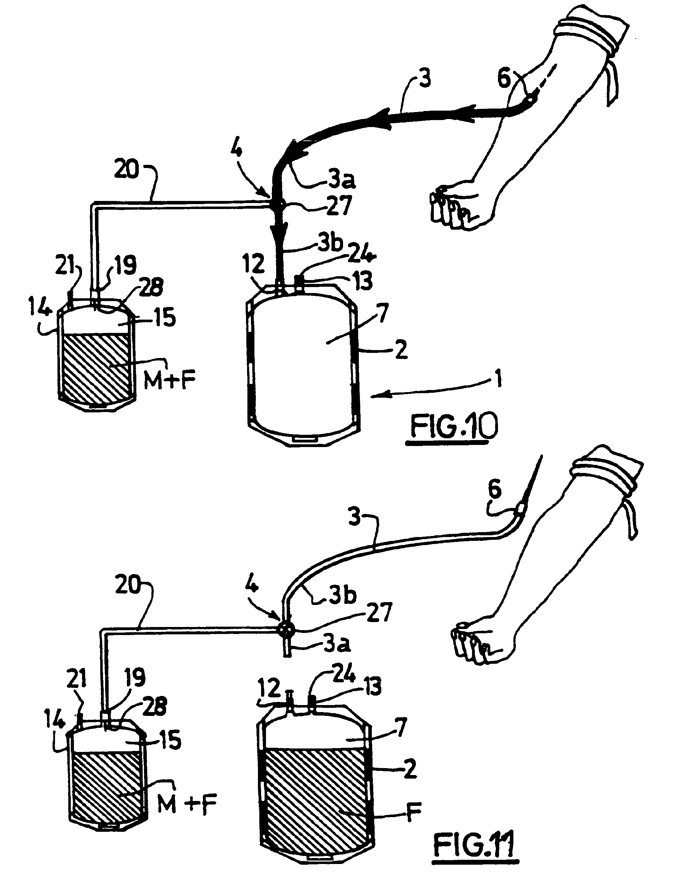

Les figures 8 à 11 sont des vues schématiques en élévation et à plat illustrant les étapes successives d'un mode d'exécution du procédé de mise en oeuvre du deuxième mode de réalisation représenté sur la figure 2. A savoir, pour la figure 9, l'étape de prélèvement du fluide biologique à l'aide de l'ensemble de prélèvement ou d'amenée ; pour la figure 10, l'alimentation de la poche test en fluide biologique ; pour la figure 11 , une quantité suffisante de fluide biologique ayant été introduite dans la poche test, les moyens de dérivation et/ou d'arrêt sont actionnés pour permettre l'alimentation de la poche du système à poche(s) seule ; pour la figure 12, la quantité souhaitée de fluide biologique ayant été introduite dans la poche du système à poche(s), arrêt du prélèvement du fluide biologique.

Un système à poche(s) 1 tel que ceux destinés à recevoir un fluide biologique F comprend typiquement une poche 2 à usage médical sur laquelle est branchée une ou plusieurs tubulures -notamment au moins une tubulure principale 3-, des moyens de dérivation et/ou d'arrêt associés 4 à la ou aux tubulures, et un ensemble de prélèvement ou d'amenée 5 du fluide biologique F.

La poche 2 est destinée à recevoir un fluide biologique F tel que du sang ou un composant du sang provenant de l'ensemble de prélèvement ou d'amenée 5 du fluide biologique F par l'intermédiaire de la tubulure principale 3. Le fluide biologique F peut soit être prélevé directement sur un donneur soit provenir d'une autre poche à usage médical soit amené autrement.

L'ensemble de prélèvement ou d'amenée 5 du fluide biologique F, qui ne sera pas décrit plus avant, peut comprendre, par exemple, une aiguille de prélèvement 6 à introduire dans une veine d'un donneur ou encore une machine de prélèvement.

Un tel système à poche(s) 1 s'applique typiquement au prélèvement du sang d'un donneur au moyen d'une aiguille de prélèvement 6 connecté à la tubulure principale 3, le sang étant alors envoyé vers et reçu dans la poche 2 du système à poche(s) 1.

Le système à poche(s) 1 peut comprendre en outre et selon les configurations et usages souhaités, par exemple une ou plusieurs autres poches à usage médical, un ou plusieurs filtres ou clamps associés entre eux par une ou plusieurs tubulures. Ces différentes configurations ne sont pas décrites plus avant, dans la mesure ou elles comprennent le système à poche(s) 1 selon la structure ici décrite.

La poche 2 comprend une enveloppe 7 sur laquelle est montée au moins un site assurant la communication fluidique avec l'extérieur de l'enveloppe 7. L'enveloppe 7 se présente sous forme de tube aplati ou de feuille en matériau plastique souple dont les parties extrêmes transversales et/ou longitudinales ont été soudées respectivement transversalement ou longitudinalement. L'enveloppe 7 est destinée à contenir le fluide biologique F.

Dans les réalisations particulières de poche 2 représentées sur les figures, l'enveloppe 7 comporte d'un même coté deux orifices 8, 9 de forme générale cylindrique. Dans chacun de ces orifices 8, 9 est monté de façon étanche une portion de tube 10, 11 qui fait saillie de l'enveloppe 7 afin d'assurer la communication fluidique avec l'extérieur de l'enveloppe 7. Cet agencement permet de former deux sites 12, 13 dans lesquels peuvent être introduit de façon étanche des tubulures ou tout autre dispositif permettant de récupérer et/ou d'introduire un fluide dans l'enveloppe 7.

Dans un de ces sites 12 est monté de façon étanche la tubulure principale 3. L'ensemble de prélèvement ou d'amenée 5 du fluide biologique F est alors associé à et branché sur la tubulure principale 3, à l'opposé de la poche 2.

Un système à poche(s) 1 tel qu'il vient d'être décrit peut faire l'objet de nombreuses variantes de réalisation plus ou moins complexes. Ses usages peuvent être variés. Par exemple, il permet de prélever du sang d'un donneur, la poche 2 contenant le sang prélevé étant ensuite stockée, manipulée et traitée comme telle par exemple pour séparer le sang en ses divers composants.

Au moins une autre poche à usage médical - dite poche test 14 - est appliquée au système à poche(s) 1 tel qu'il vient d'être décrit grâce à une ou à un système de tubulures 20 pour former avec le système à poche(s) 1, la tubulure principale 3 et l'ensemble de prélèvement ou d'amenée 5 un ensemble clos et unitaire.

Un test approprié de stérilité/contamination du fluide biologique F ayant à être reçu dans la poche 2 est réalisé dans la poche test 14, spécialement destinée à cette fin.

On comprend, dès lors, que la poche test 14, bien que formant à l'origine avec le système à poche(s) 1 un ensemble unitaire, ne fait pas directement partie in fine du système à poche(s) 1 en vue du stockage, de la manipulation et du traitement dont il a été question plus haut.

La poche test d'une part contient un milieu de culture ou un réactif M apte à mettre en évidence une éventuelle contamination du fluide biologique F - à contrario à mettre en évidence sa stérilité - et d'autre part est destinée à recevoir une certaine quantité appropriée de fluide biologique F, afin de permettre une éventuelle réaction entre eux en cas de contamination du fluide biologique F.

La poche test 14 est formée par une enveloppe 15 sur laquelle est montée au moins un site assurant la communication fluidique avec l'extérieur de l'enveloppe 15. L'enveloppe 15 se présente, dans une réalisation, de manière analogue aux poches à usage médical sous forme de tube aplati ou de feuille en matériau plastique souple dont les parties extrêmes transversales et/ou longitudinales ont été soudées respectivement transversalement ou longitudinalement. L'enveloppe 15 est destinée à recevoir le milieu de culture ou le réactif M et ensuite additionnellement une certaine quantité de fluide biologique F.

Dans la réalisation qui vient d'être décrite et dans laquelle la poche test 14 prend la forme d'une poche à usage médical, l'ensemble système à poche(s) 1 / poche test 14 est de nature homogène. Toutefois, dans une autre réalisation, la poche test 14 peut être réalisée différemment, par exemple sous la forme d'un récipient rigide ou semi rigide tel qu'un petit flacon. Il est entendu toutefois que l'expression « poche test » utilisée vise toutes les formes de conteneur, souples tel que poche, rigides tel que flacon ou autres.

Dans les réalisations particulières de poches test 14 représentées sur les figures, l'enveloppe 15 comporte deux orifices 16, 17 de forme générale cylindrique agencés sur un coté de l'enveloppe 15.

Dans un premier orifice 16 est monté de façon étanche une portion de tube 18 munie d'un ouvre-circuit 28 afin d'isoler, avant sa rupture, le milieu de culture ou le réactif M contenu dans la poche test 14 du système à poche(s) 1. La portion de tube 18 fait saillie de l'enveloppe 15 afin d'assurer la communication fluidique avec l'extérieur de l'enveloppe 15. Cet agencement permet de former un site 19 dans lequel est introduit de façon étanche une tubulure 20 qui permet d'associer la poche test 14 au système à poche(s) 1 afin de réaliser l'alimentation de la poche test 14 en fluide biologique F.

Dans le deuxième orifice 17 est monté de façon étanche un site d'injection 21 permettant l'injection du milieu de culture ou du réactif M dans la poche test 14 au moyen d'une aiguille 22 et d'une seringue d'injection 23 (voir figure 3). Le site d'injection 21 est par exemple formé par un tampon en élastomère monté dans un embout creux. Le tampon en élastomère étant prévu pour être traversé par l'aiguille d'injection 22 et se refermer de lui-même une fois l'aiguille 22 enlevée.

Dans d'autres modes d'introduction du milieu de culture ou du réactif M dans la poche test 14, le milieu de culture ou le réactif M est introduit dans la poche test 14 par un autre moyen qu'une aiguille 22 et une seringue d'injection 23. Par exemple, l'orifice 17 ne comprend pas de site d'injection 21 mais une tubulure au moyen de laquelle le milieu de culture ou le réactif M est introduit dans la poche test 14. Une fois l'introduction réalisée, la tubulure est alors fermée par un bouchon ou soudée.

Dans une autre réalisation de la poche test 14, il n'est pas prévu un tel deuxième orifice 17, la poche test 14 étant remplie de fabrication avec le milieu de culture ou le réactif M. On sait en effet qu'il existe des techniques permettant de fabriquer des sachets - tels que la poche test 14 - et de les remplir au moment de la fabrication - notamment au dernier soudage transversal - avec un certain contenu liquide - ou autre -.

La poche test 14 étant prévue pour contenir la quantité de milieu de culture ou de réactif M nécessaire - et généralement suffisante - pour le test de contamination, par exemple de l'ordre de quelques millilitres de milieu de culture ou de réactif M et la quantité de fluide biologique F nécessaire - et généralement suffisante - pour le test de contamination, par exemple de l'ordre de quelques millilitres de fluide biologique F, la contenance de la poche test 14 est inférieure à celle de la poche 2, par exemple de l'ordre de 25 fois inférieure. Toutefois, la poche test 14 et la poche 2 peuvent être de contenance identique ou voisine et/ou comporter un autre nombre d'orifices agencés différemment sur leur enveloppe 7, 15 respective.

Dans une première variante de réalisation d'un ensemble comprenant la poche test 14 et le milieu de culture ou le réactif M qu'elle contient, l'injection du milieu de culture ou du réactif M dans la poche test 14 est réalisée avant l'association de la poche test 14 au système à poche(s) 1. C'est alors l'ensemble formé de la poche test 14 et du milieu de culture ou du réactif M qu'elle contient qui est associé au système à poche(s) 1 par l'intermédiaire d'une ou d'un système de tubulure 20.

Dans une seconde variante de réalisation d'un ensemble comprenant la poche test 14 et le milieu de culture ou le réactif M qu'elle contient, la poche test 14 vide est associée au système à poche(s) 1 au moyen d'une ou d'un système de tubulure 20 pour former avec la tubulure principale 3, la poche 2 et l'ensemble de prélèvement ou d'amenée 5 un ensemble unitaire et clos. Le milieu de culture ou le réactif M est ensuite introduit dans la poche test 14 de cet ensemble, par exemple préalablement à l'alimentation en fluide biologique F.

Bien que sur les figures 4 à 11, la poche test 14 contienne le milieu de culture ou le réactif M avant l'introduction du fluide biologique F, la réalisation suivant laquelle on introduit d'abord du fluide biologique F dans la poche test 14 puis le milieu de culture ou le réactif M pour effectuer le test de contamination est également envisageable.

Nous allons maintenant décrire, en relation avec les figures 1 et 2 et 4 à 11, un premier et un deuxième mode de réalisation d'un ensemble comprenant un système à poche(s) 1 et une poche test 14 ainsi qu'un mode d'exécution d'un procédé de mise en oeuvre de chacun de ces modes de réalisation.

Ces deux modes de réalisation illustrent le cas où une seule poche test 14 est associée à la poche 2 pour la réalisation d'un test de contamination. Toutefois, l'association de plusieurs poches test 14 sur la poche 2 est également envisageable. Les milieux de culture M reçus dans chacune de ces poches test 14 peuvent alors être différents ou identiques. De telles configurations permettent d'effectuer plusieurs tests de contamination différents, par exemple pour tester plusieurs contaminants, et/ou identiques, par exemple pour améliorer la fiabilité du test. Ces tests de contamination pouvant être effectués simultanément ou à des instants différents, par exemple en début puis en fin de prélèvement.

Les flèches sur les tubulures 3, 20 qui ont été dessinées sur les figures 4 à 11 illustrent l'écoulement du fluide biologique F respectivement du donneur vers la poche 2, du donneur vers la poche test 14 ou de la poche 2 vers la poche test 14.

Dans les modes de réalisation d'un ensemble comprenant un système à poche(s) et une poche test représentés sur les figures 1, 2 et 4 à 11, la tubulure 20 de la poche test 14 est connectée à la tubulure principale 3 du système à poche(s) 1 pour former avec l'ensemble de prélèvement ou d'amenée 5 un ensemble clos et unitaire. Cette configuration permet d'alimenter la poche test 14 avec du fluide biologique F qui a la même origine que celui ayant à être reçu dans la poche 2 du système à poche(s) 1, tout en étant différent de lui pour ne pas provenir du système à poche(s) 1.

Des moyens de dérivation et/ou d'arrêt 4 du fluide biologique F commandés par des moyens d'actionnement sont prévus pour permettre de diriger sélectivement du fluide biologique F vers la poche test 14 et/ou vers la poche 2 du système à poche(s) 1.

Dans cet agencement, le site 13 de la poche 2 du système à poche(s) 1 dans lequel la tubulure principale 3 n'est pas branchée est obstrué, par exemple par un bouchon 24 ou par une languette munie d'un tube operculé.

Dans le première mode de réalisation d'un ensemble comprenant un système à poche(s) et une poche test pour la réalisation d'un test de contamination, représenté plus particulièrement sur les figures 1 et 4 à 7, la connexion entre la tubulure principale 3 et la tubulure 20 de la poche test 14 est réalisée par une jonction 25, par exemple en T, mettant en communication l'ensemble de prélèvement ou d'amenée 5 du fluide biologique F d'une part avec la poche test 14 et d'autre part avec la poche 2. La jonction 25 peut, par exemple, scinder la tubulure principale 3 en deux parties respectivement amont 3a et aval 3b par rapport à l'écoulement du fluide biologique F du donneur vers la poche 2. Les moyens de dérivation et/ou d'arrêt 4 du fluide biologique F sont alors réalisés à l'aide d'un clamp 26 prévu sur la tubulure 20 de la poche test 14 et d'un clamp 29 prévu sur la partie de tubulure principale 3b (voir figure 1). Après avoir cassé l'ouvre-circuit 28, le clamp 26 permet de mettre en et hors communication fluidique la poche test 14 avec la tubulure principale 3. La tubulure 20 peut être équipée d'un clapet anti-retour afin de s'assurer que le milieu de culture ou le réactif M ne puisse passer dans le fluide biologique F contenu dans la poche 2. Cet arrangement permet d'éviter toute erreur de manipulation. Le clamp 29 permet de mettre en et hors communication fluidique la poche 2 avec la tubulure principale 3. Ces deux configurations respectives permettent de définir respectivement les termes clamp 26, 29 ouvert et clamp 26, 29 fermé.

Lors du prélèvement du fluide biologique F, par exemple à l'aide d'une aiguille de prélèvement 6 introduit dans une veine d'un donneur, le fluide biologique F est amené jusqu'à la jonction 25 par la partie amont 3a de la tubulure principale 3 (voir figure 4). Le fluide biologique F peut ensuite alimenter d'une part la poche 2 par l'intermédiaire de la partie aval 3b de la tubulure principale 3 et d'autre part la poche test 14 via les moyens de dérivation et/ou d'arrêt 4.

Dans l'étape d'exécution du procédé de mise en oeuvre de ce premier mode de réalisation, représentée sur la figure 4, les clamps 26, 29 sont fermés lors de l'introduction d'une aiguille de prélèvement 6 dans la veine du donneur. Pour effectuer le test de stérilité/contamination du fluide biologique F, l'ouvre-circuit 28 est rompu et le clamp 26 est manoeuvré pour permettre l'alimentation en fluide biologique F de la poche test 14 par l'intermédiaire de la tubulure 20 associant la poche test 14 au système à poche(s) 1 (voir figure 5).

Une fois qu'une quantité suffisante de fluide biologique F a été introduite dans la poche test 14, le clamp 26 est de nouveau manoeuvré pour fermer l'alimentation de la poche test 14 en fluide biologique F et le clamp 29 est ouvert (voir figure 6). Sur cette figure est représentée une étape d'exécution du procédé de mise en oeuvre suivant laquelle la poche test 14 est alors dissociée de l'ensemble formé de la poche 2, de la tubulure principale 3 et de l'ensemble de prélèvement ou d'amenée 5, par exemple en coupant et soudant la tubulure 20 en aval du clamp 26 fermé. La poche test 14 peut alors subir des traitements tels qu'agitation ou chauffage dans le but d'améliorer la réaction entre le milieu de culture ou le réactif M et le fluide biologique F et ainsi de mettre en évidence une éventuelle contamination du fluide biologique F.

Le clamp 26 étant fermé et le clamp 29 ouvert, le prélèvement du fluide biologique F est poursuivi de façon conventionnelle jusqu'à ce que la quantité souhaitée de fluide biologique F ait été prélevé du donneur pour être dirigée vers et reçu dans la poche 2 du système à poche(s) 1. L'aiguille de prélèvement 6 pouvant alors être retiré du bras du donneur (voir figure 7).

Dans le mode d'exécution du procédé de mise en oeuvre du premier mode de réalisation, représenté plus particulièrement sur les figures 4 à 7., l'alimentation de la poche test 14 et de la poche 2 est réalisée de façon séquentielle et notamment l'alimentation de la poche test 14 est réalisée avant celle de la poche 2. Par exemple, l'alimentation de la poche 2 peut ainsi être effectuée à la condition que le test de contamination du fluide biologique F soit négatif, c'est à dire que le fluide biologique F se soit révélé stérile.

Dans une variante (non représentée) du premier mode de réalisation d'un ensemble comprenant un système à poche(s) 1 et une poche test 14, le fluide biologique F peut être dirigé en continu vers la poche 2 et notamment en même temps que vers la poche test 14.

Dans le deuxième mode de réalisation d'un ensemble comprenant un système à poche(s) 1 et une poche test 14 pour la réalisation d'un test de contamination, représentée plus particulièrement sur les figures 2 et 8 à 11, la connexion entre la tubulure 20 de la poche test 14 et la tubulure principale 3 est réalisée par un robinet à voies multiples 27 prévu pour mettre en communication l'ensemble de prélèvement ou d'amenée 5 du fluide biologique F d'une part avec la poche test 14 et d'autre part avec la poche 2. Cet agencement permettant de former entre la poche test 14, la poche 2, les tubulures 3, 20 et l'ensemble de prélèvement ou d'amenée 5 un ensemble clos et unitaire. Le robinet à voies multiples 27 scindant la tubulure principale 3 en deux parties respectivement amont 3a et aval 3b par rapport à l'écoulement du fluide biologique F du donneur vers la poche 2 (voir figure 2). Ce robinet à voies multiples 27 formant également moyen de dérivation et/ou d'arrêt 4 du fluide biologique F.

Lors du prélèvement du fluide biologique F par exemple à l'aide d'une aiguille de prélèvement 6 introduit dans une veine d'un donneur, le fluide biologique F est amené jusqu'au robinet à voies multiples 27 par l'intermédiaire de la partie amont 3a de la tubulure principale 3 (voir figure 8).

Dans le deuxième mode de réalisation d'un ensemble comprenant un système à poche(s) 1 et une poche test 14 représenté sur les figures 8 à 11, le robinet à voies multiples 27 est tel qu'il permet l'alimentation de la poche test 14 ou de la poche 2 de façon séquentielle et notamment, l'alimentation de la poche test 14 avant celle de la poche 2.

Pour effectuer le test de stérilité/contamination du fluide biologique F , l'ouvre-circuit 28 est rompu et le robinet à voies multiples 27 est manoeuvré pour permettre l'alimentation de la poche test 14 en fluide biologique F alors que celle de la poche 2 est fermée (voir figure 9).

Une fois qu'une quantité suffisante de fluide biologique F a été introduite dans la poche test 14, le robinet à voies multiples 27 est de nouveau actionné pour fermer l'alimentation de la poche test 14 en fluide biologique F et ouvrir celle de la poche 2 de façon simultanée (voir figure 10). Le milieu de culture ou le réactif M et le fluide biologique F peuvent alors réagir pour mettre en évidence une éventuelle contamination du fluide biologique F. Dans une variante d'exécution du procédé de mise en oeuvre, l'ouverture de l'alimentation en fluide biologique F de la poche 2 peut ne pas se faire simultanément à la fermeture de celle de la poche test 14 mais, par exemple après que le test de contamination se soit révélé négatif.

A la fin du prélèvement, le robinet à voies multiples 27 est manoeuvré pour fermer la communication entre l'ensemble de prélèvement ou d'amenée 5 et la poche 2 puis l'aiguille de prélèvement 6 est retiré du bras du donneur. Dans l'étape d'exécution du procédé de mise en oeuvre représentée sur la figure 11, la poche 2 est ensuite désolidarisée de l'ensemble clos et unitaire, par exemple en coupant et soudant la tubulure principale 3 en aval du robinet à voies multiples 27. La poche 2 contenant le fluide biologique F pouvant alors être manipulée indépendamment du reste de l'ensemble, par exemple en vue de son stockage.

Dans une variante (non représentée) du deuxième mode de réalisation d'un ensemble comprenant un système à poche(s) 1 et une poche test 14, le robinet à voies multiples 27 est tel qu'il permet l'alimentation sélective soit de la poche 2 et de la poche test 14, soit de la poche 2 seule.

Pour effectuer le test de stérilité/contamination du fluide biologique F, l'ouvre-circuit 28 est rompu et le robinet à voies multiples 27 est alors actionné pour ouvrir l'alimentation en fluide biologique F de la poche test 14 et celle de la poche 2 de façon simultanée. L'écoulement du milieu de culture ou du réactif M de la poche test 14 dans la tubulure principale 3 du système à poche(s) 1 est rendu impossible.

Une fois qu'une quantité suffisante de fluide biologique F a été introduite dans la poche test 14, le robinet à voies multiples 27 est de nouveau actionné pour fermer l'alimentation en fluide biologique F de la poche test 14 en gardant ouvert celle de la poche 2. Le milieu de culture ou le réactif M et le fluide biologique F peuvent alors réagir à l'intérieur de la poche test 14 pour mettre en évidence une éventuelle contamination du fluide biologique F.

A la fin du prélèvement, le robinet à voies multiples 27 est manoeuvré pour fermer la communication entre l'ensemble de prélèvement ou d'amenée 5 et la poche 2 en laissant fermer la communication entre l'ensemble de prélèvement ou d'amenée 5 et la poche test 14, puis l'aiguille de prélèvement 6 est retiré du bras du donneur.

## Revendications

1. Ensemble système à poche(s) (1) / poche test (14) comprenant:
- un système à poche(s) (1) destiné à recevoir un fluide biologique (F) et comprenant au moins une poche (2) sur laquelle est branchée au moins une tubulure principale (3), et un ensemble de prélèvement ou d'amenée (5) du fluide biologique (F) branché sur ladite tubulure principale (3), et
- au moins une poche test (14) contenant un milieu de culture ou un réactif (M) qui recevant du fluide biologique (F) est apte à mettre en évidence une éventuelle contamination,
**caractérisé en ce que** le système à poche(s) (1) comprend en outré des moyens de dérivation et/ou d'arrêt (4) associés à ladite tubulure principale (3), et **en ce que** la poche test (14) est reliée ou destinée à être reliée à la poche (2) par l'intermédiaire d'au moins ladite tubulure principale (3).

2. Ensemble selon la revendication 1, selon lequel la poche test (14) est apte à contenir une quantité suffisante de milieu de culture ou de réactif (M) et de fluide biologique (F) pour la réalisation du test de contamination, par exemple de l'ordre de quelques millilitres de milieu de culture ou de réactif (M) et de l'ordre de quelques millilitres de fluide biologique (F).

3. Ensemble selon la revendication 1 ou 2, selon lequel la poche test (14) est reliée ou est destinée à être reliée à la tubulure principale (3) par l'intermédiaire d'une ou d'un système de tubulures (20).

4. Ensemble selon l'une quelconque des revendications 1 à 3, selon lequel les moyens de dérivation et/ou d'arrêt (4) comprennent une jonction (25) et/ou un clamp (26,29) prévu sur la tubulure principale (3).

5. Ensemble selon la revendication 4 lorsqu'elle dépend de la revendication 3, selon lequel les moyens de dérivations et/ou d'arrêt (4) comprennent en outre un clamp sur la ou le système de tubulures (20).

6. Ensemble selon l'une quelconque des revendications 1 à 3, selon lequel les moyens de dérivation et/ou d'arrêt (4) comprennent un robinet à voie multiple (27).

7. Ensemble selon l'une quelconque des revendications 1 à 6, formant un ensemble clos et unitaire.

8. Procédé de mise en oeuvre d'un ensemble système à poche(s) / poche test selon la revendication 1, dans lequel :
- on prélève le fluide biologique (F) à l'aide de l'ensemble de prélèvement ou d'amenée (5) ;
- on dirige du fluide biologique (F) vers la poche test (14) jusqu'à ce qu'une quantité suffisante au test de contamination y soit introduite;
- on actionne les moyens de dérivation et/ou d'arrêt (4) pour fermer l'alimentation en fluide biologique (F) de la poche test (14) ;
- on dirige le fluide biologique (F) vers la poche (2) du système à poche(s) (1) par l'intermédiaire de la tubulure principale (3).

9. Procédé selon la revendication 8, dans lequel on dirige du fluide biologique (F) vers la poche test (14) en même temps que vers la poche (2) du système à poche(s) (1).

10. Procédé selon la revendication 8, dans lequel on dirige du fluide biologique (F) vers la poche test (14) avant que vers la poche (2) du système à poche(s) (1).

11. Procédé selon l'une quelconque des revendications 8 à 10, dans lequel on prélève une quantité de fluide biologique (F) suffisante pour la réalisation du test de contamination, par exemple de l'ordre de quelques millilitres de fluide biologique (F), pour l'introduire dans la poche test (14).

## Claims

1. A bag system (1)/test bag (14) assembly, comprising:
- a bag system (1) for receiving a biological fluid (F) and comprising at least one bag (2) to which at least one main tube (3) is connected, and an assembly (5) for drawing or conveying biological fluid (F) branched from said main tube (3), and _
- at least one test bag (14) containing a culture medium or a reagent (M) which, upon receiving the biological fluid (F), is able to show any contamination,
**characterised in that** the bag system (1) further comprises bypass and/or stop means (4) associated with said main tube (3), and **in that** the test bag (14) is connected or is intended for connection to the bag (2) via at least said main tube (3).

2. The assembly according to claim 1, wherein the test bag (14) is able to contain a sufficient amount of culture or reagent medium (M) and biological fluid (F) to carry out the contamination test, for example approximately a few millilitres of culture or reagent medium (M) and approximately a few millilitres of biological fluid (F).

3. The assembly according to either claim 1 or 2, wherein the test bag (14) is connected or is intended for connection to the main tube (3) via a tube or a system of tubes (20).

4. The assembly according to any one of claims 1 to 3, wherein the bypass and/or stop means (4) comprise a junction (25) and/or a clamp (26, 29) provided on the main tube (3).

5. The assembly according to claim 4, when dependent on claim 3, wherein the bypass and/or stop means (4) further comprise a clamp on the tube or system of tubes (20).

6. The assembly according to any one of claims 1 to 3, wherein the bypass and/or stop means (4) comprise a multi-way valve (27).

7. The assembly according to any one of claims 1 to 6, forming a closed and unitary assembly.

8. A method for implementing a bag system/test bag assembly according to claim 1, wherein:
- the biological fluid (F) is drawn with the aid of the drawing or conveying assembly (5);
- the biological fluid (F) is directed towards the test bag (14) until an amount sufficient for the contamination test has been introduced;
- the bypass and/or stop means (4) are actuated to close the supply of biological fluid (F) to the test bag (14);
- the biological fluid (F) is directed towards the bag (2) of the bag system (1) via the main tube (3).

9. The method according to claim 8, wherein biological fluid (F) is directed towards the test bag (14) at the same time as towards the bag (2) of the bag system (1).

10. The method according to claim 8, wherein biological fluid (F) is directed towards the test bag (14) before being directed towards the bag (2) of the bag system (1).

11. The method according to any one of claims 8 to 10, wherein an amount of biological fluid (F) sufficient to carry out the contamination test is drawn, for example approximately a few millilitres of biological fluid (F), for introduction into the test bag (14).

## Patentansprüche

1. Satz aus Beutelsystem (1) und Testbeutel (14), umfassend:
- ein Beutelsystem (1), das dazu bestimmt ist, ein biologisches Fluid (F) aufzunehmen und das mindestens einen Beutel (2) umfasst, an den mindestens eine Hauptschlauchleitung (3) angeschlossen ist, und einen Satz zur Entnahme oder zum Transport (5) des biologischen Fluids (F), der an der Hauptschlauchleitung (3) angeschlossen ist, und
- mindestens einen Testbeutel (14), der ein Kulturmedium oder ein Reagens (M) enthält, das beim Aufnehmen des biologischen Fluids (F) in der Lage ist, eine mögliche Kontamination nachzuweisen,
**dadurch gekennzeichnet, dass** das Beutelsystem (1) ferner Ableitungsmittel und/oder Stoppmittel (4) umfasst, die mit der Hauptschlauchleitung (3) assoziiert sind, und dadurch, dass der Testbeutel (14) mittels mindestens der Hauptschlauleitung (3) mit dem Beutel (2) verbunden ist oder dazu bestimmt ist, mit diesem verbunden zu werden.

2. Satz nach Anspruch 1, wobei der Testbeutel (14) in der Lage ist, eine ausreichende Menge an Kulturmedium oder Reagens (M) und biologischem Fluid (F) für die Durchführung des Kontaminationstest zu enthalten, zum Beispiel in der Größenordnung von einigen Millilitern Kulturmedium oder Reagens (M) und in der Größenordnung von einigen Millilitern biologischem Fluid (F).

3. Satz nach Anspruch 1 oder 2, wobei der Testbeutel (14) mit der Hauptschlauchleitung (3) mittels einer Schlauchleitung oder eines Schlauchleitungssystems (20) verbunden ist oder dazu bestimmt ist, damit verbunden zu werden.

4. Satz nach einem der Ansprüche 1 bis 3, wobei die Ableitungsmittel und/oder die Stoppmittel (4) ein Anschlussstück (25) und/oder eine Klemme (26, 29) umfassen, die auf der Hauptschlauchleitung (3) vorgesehen ist.

5. Satz nach Anspruch 4, sofern er von Anspruch 3 abhängig ist, wobei die Ableitungsmittel und/oder die Stoppmittel (4) ferner eine Klemme auf der Schlauchleitung oder auf dem Schlauchleitungssystem (20) umfassen.

6. Satz nach einem der Ansprüche 1 bis 3, wobei die Ableitungs- und/oder Stoppmittel (4) einen Mehrwegehahn (27) umfassen.

7. Satz nach einem der Ansprüche 1 bis 6, der einen Satz bildet, der geschlossen und eine Einheit ist.

8. Verfahren zum Einsetzen eines Satzes aus Beutelsystem und Testbeutel nach Anspruch 1, wobei:
- das biologische Fluid (F) mithilfe des Satzes zur Entnahme oder zum Transport (5) entnommen wird;
- biologisches Fluid (F) zum Testbeutel (14) geleitet wird, bis dort eine für den Kontaminationstest ausreichende Menge eingeführt ist;
- die Ableitungs- und/oder Stoppmittel (4) zum Beenden der Versorgung des Testbeutels (14) mit biologischem Fluid (F) betätigt werden;
- das biologische Fluid (F) mittels der Hauptschlauchleitung (3) zum Beutel (2) des Beutelsystems (1) gelenkt wird.

9. Verfahren nach Anspruch 8, wobei biologisches Fluid (F) gleichzeitig zum Testbeutel (14) und zum Beutel (2) des Beutelsystems (1) gelenkt wird.

10. Verfahren nach Anspruch 8, wobei biologisches Fluid (F) zum Testbeutel (14) gelenkt wird, bevor es zum Beutel (2) des Beutelsystems (1) gelenkt wird.

11. Verfahren nach einem der Ansprüche 8 bis 10, wobei eine Menge an biologischem Fluid (F) entnommen wird, die für die Durchführung des Kontaminationstests ausreichend ist, zum Beispiel in der Größenordnung von einigen Millilitern an biologischem Fluid (F), um sie in den Testbeutel (14) einzuführen.
